# FASCICULE DE BREVET EUROPEEN

(11) **EP 1 984 031 B1**
(45) Date de publication et mention de la délivrance du brevet: **27.07.2011**
(21) Numéro de dépôt: 07730997.9
(22) Date de dépôt: 16.02.2007
(51) Int. Cl.: A61L 24/00, A61F 5/448

(54) **RACCORD POUR POCHE DE STOMIE , DU TYPE DEUX PIÈCES , FIXÉ PAR COLLAGE**
ANSCHLUSSSTÜCK FÜR STOMABEUTEL DER ZWEITEILIGEN ART, DAS DURCH KLEBEN BEFESTIGT WIRD
CONNECTOR FOR STOMA BAG, OF THE TWO-PIECE TYPE, FIXED BY GLUING

(30) Priorité: 17.02.2006 FR 0601424
(43) Date de publication de la demande: 29.10.2008
(73) Titulaire: B.Braun Medical SAS, 92100 Boulogne-Billancourt (FR)
(72) Inventeur: BURLOT, Delphine, F-64600 Anglet (FR); LASSALLE, Paul, F-64200 BIARRITZ (FR)
(74) Mandataire: Lebrette, Camille
(86) Numéro de dépôt international: PCT/FR2007/000286
(87) Numéro de publication internationale: WO 2007/093718

(56) Documents cités:
- WO-A-01/85074
- US-A- 5 429 625
- US-A1- 2005 177 119

## Description

La présente invention concerne un appareillage de stomie du type deux pièces. Plus précisément, l'invention concerne un raccord adhésif d'un tel appareillage.

Un raccord adhésif d'appareillage de stomie .du type deux pièces comprend d'une part un support muni d'un étrier de fixation à la peau d'un patient et d'une piste de collage, et d'autre part une poche munie d'une piste de collage portant un adhésif. Ces raccords collés permettent l'utilisation d'un même support avec plusieurs poches successives, sans irriter la peau du patient par un retrait fréquent du support. Comme l'adhésif qui fixe la poche au support est l'élément d'interface, il constitue l'élément le plus fragile et, pour cette raison, il est toujours placé sur la poche, qui est l'élément à usage unique et de courte durée, et non sur le support, qui est un élément relativement fixe, utilisé avec plusieurs poches les unes après les autres.

Dans ces appareillages de type deux pièces à raccord collé, l'adhésif placé entre les pistes du support et de la poche, pour ne pas rester collé sur la piste du support lors du retrait de la poche, présente toujours une plus grande adhésivité à la piste de la poche qu'à la piste du support.

L'utilisation d'un tel appareillage de stomie de type deux pièces comprend donc la mise en oeuvre de deux interfaces collées : une première interface se trouve entre le support et la peau du patient, et l'autre entre les pistes du support et de la poche.

Ces interfaces collées comprennent des adhésifs qui sont tous deux du type "structurel". Un adhésif "structurel" est tel que les parties collées supportent une charge au moins égale à leur poids. Ces adhésifs se distinguent des adhésifs de retenue qui ne sont pas soumis en permanence à des forces d'arrachement, et des adhésifs de colmatage dont le rôle est essentiellement une obturation. Dans le cas des appareillages de stomie, lorsqu'une poche contient des matières évacuées, son poids est bien supérieur à celui des parties collées par un adhésif qui doit donc être de type structurel.

On sait que les adhésifs structurels résistent d'autant mieux que leur épaisseur est faible, compte tenu des limites fixées par l'application. En effet, une trop grande épaisseur du film ou de la couche d'adhésif réduit la force de retenue ; cependant, il est nécessaire que le film ou la couche ne soit pas trop mince, car il peut alors provoquer un collage discontinu. Dans le cas des appareillages de stomie, toute discontinuité de collage entraîne des fuites des matières évacuées, notamment des liquides et des gaz (mauvaises odeurs).

Les couches adhésives des appareillages de stomie doivent avoir une résistance au cisaillement particulièrement élevée car, en position debout du patient, tout le poids de la poche s'exerce dans le plan des surfaces de collage.

De manière générale, on considère que, dans les joints collés, l'adhésif structurel doit avoir une épaisseur comprise entre 0,1 et 0,43 mm (4 à 13 millièmes de pouce). Pour cette raison, on utilise, pour le collage des pistes du support et de la poche, un adhésif structurel de faible épaisseur, de l'ordre de 0,1 à 0,3 mm en général.

Cependant, la couche d'adhésif structurel placée entre le support et la peau du patient a une plus grande épaisseur, par exemple de 1 à 2 mm. En effet, l'utilisation d'un simple adhésif au contact de la peau pose des problèmes de tolérance cutanée, de sorte qu'on ne peut pas utiliser un "simple" adhésif. Le matériau utilisé d'une part doit être déformable, compte tenu des mouvements de la surface de la peau (une couche rigide réduit beaucoup le confort du patient) dus aux mouvements du patient, mais doit aussi avoir des propriétés non irritantes au contact de la peau. Pour cette raison, on utilise essentiellement des couches de "gomme" chargée en hydrocolloïdes. La résistance "structurelle" d'une telle couche est obtenue par l'utilisation d'un matériau à propriétés élastomères.

La couche d'adhésif structurel placée entre le support et la peau du patient est donc spécialement adaptée aux conditions fixées par cette application : la déformation de la peau et, de façon plus générale, de la partie de corps entourant une stomie lorsque le patient effectue divers mouvements, et l'absence d'irritation de la peau. Ces conditions sont remplies lors de l'utilisation d'une couche de "gomme" à propriétés élastomères chargée en hydrocolloïdes, ayant une épaisseur de l'ordre de un à deux millimètres.

Dans les appareillages de stomie du type deux pièces à raccord collé concernés, la couche de gomme, le plus souvent collée à une feuille de support (cette couche et la feuille, avec parfois une feuille protectrice de la gomme, étant parfois appelées "étrier" ou "protecteur cutané"), est en général fixée à la piste du support par une simple soudure circulaire de faible largeur entre la feuille de support et la piste. De cette manière, la piste du support est relativement indépendante de l'étrier et la piste de la poche peut ainsi être facilement collée sur la piste du support.

Cependant, on s'est déjà rendu compte, comme décrit par exemple dans le document WO 96/38 106, qu'il se forme parfois des plis entre les pistes du support et de la poche, notamment lorsque le patient qui porte l'appareillage se baisse. On conçoit que de tels plis, s'ils conduisent à la formation de canaux, c'est-à-dire à des décollements plus ou moins fins suivant une ou plusieurs lignes de pli, entraînent presque nécessairement des fuites non seulement de gaz et d'odeurs dans le cas des poches de colostomie, mais surtout de fluides dans le cas des poches d'iléostomie et d'urostomie.

La formation de ces plis, et donc des décollements de la piste de la poche, est due au fait que la piste du support et, en conséquence, celle de la poche après collage, ne sont pas suffisamment découplées de l'étrier du support qui doit avoir une grande liberté de déformation et une grande souplesse pour suivre au mieux les mouvements du patient.

Pour la solution de ce problème, les documents WO-01/85 074 et WO-2004/039 293 décrivent diverses solutions destinées à augmenter le découplage entre la piste du support et l'étrier de celui-ci.

L'invention concerne une solution tout à fait différente du problème précité posé par la formation de plis pouvant être à l'origine de fuites.

Plus précisément, selon l'invention, la couche adhésive assurant le collage entre les pistes du support et de la poche est constituée d'une masse à propriétés élastomères suffisamment épaisse pour qu'elle encaisse les déformations au niveau d'une ou plusieurs lignes de pli et empêche en pratique les fuites entre les deux pistes.

Ainsi, l'invention concerne un raccord adhésif pour appareillage de stomie du type deux pièces, comprenant un support muni d'un étrier de fixation à la peau d'un patient et d'une piste de collage, et une poche munie d'une piste de collage portant un adhésif ; selon l'invention, l'adhésif de la piste de collage de la poche est une couche d'un adhésif structurel ayant une épaisseur d'au moins 0,5 mm, et de préférence d'au moins 0,6 mm.

De préférence, la couche d'adhésif structurel de la poche forme une masse adhésive élastomère.

De préférence, l'adhésif de la couche d'adhésif structurel contient un polymère choisi parmi les polymères d'uréthanne et les copolymères séquencés à séquences triples. Le copolymère séquencé à séquences triples est par exemple choisi parmi les copolymères styrène-isoprène-styrène, styrène-butadiène-styrène, styrène-éthylène-butadiène-styrène, et styrène-isoprène-butadiène-styrène. Le copolymère séquences peut aussi être mélangé à un polymère linéaire.

De préférence, la surface de la piste de la poche portant l'adhésif a subi un traitement d'augmentation d'adhérence. Par exemple, le traitement d'augmentation d'adhérence est un traitement chimique, tel qu'une réaction de substitution en surface, ou un traitement physique, tel qu'un traitement d'augmentation de la microrugosité, par exemple par un traitement par effluves.

Dans une variante, la couche adhésive est collée sur une feuille intermédiaire de support elle-même collée à la piste de la poche.

De préférence, le raccord comporte un organe de positionnement préalable de la piste de la poche par rapport à la piste du support. Dans un exemple, l'organe de positionnement préalable comprend des organes de coopération disposés à l'extérieur des pistes du support et de la poche. Dans un autre exemple, l'organe de positionnement préalable est disposé à l'intérieur des pistes de la poche et du support, et la couche adhésive de la piste de la poche porte une feuille protectrice formée de deux parties au moins pliées sur elles-mêmes.

Grâce aux propriétés précitées, le complexe piste du support/adhésif structurel/piste de la poche peut être plié jusqu' à un angle de 135° avant que des décollements ne se forment.

Ainsi, selon l'invention, des propriétés de déformabilité sont introduites dans la couche adhésive placée entre les deux pistes. Ces propriétés de déformabilité sont obtenues par utilisation d'un matériau particulier à propriétés élastomères, ayant bien entendu des propriétés adhésives vis-à-vis des pistes du support et de la poche.

Le matériau utilisé à cet effet, qui a des propriétés élastomères, est avantageusement un copolymère séquencé. Le copolymère séquencé peut être un copolymère de styrène-isoprène-styrène, un copolymère de styrène-butadiène-styrène, un copolymère de styrène-éthylène-butadiène-styrène, ou un copolymère de styrène-isoprène-butadiène-styrène. Ce copolymère séquencé peut être mélangé à d'autres polymères, notamment de type linéaire, par exemple un polymère de l'un des motifs du copolymère séquencé concerné. L'essentiel est que le matériau obtenu pour la couche présente des propriétés élastomères.

L'adhésif de la couche d'adhésif structurel peut aussi être une gomme à base de polyuréthanne.

Bien entendu, comme dans tout raccord pour appareillage de stomie de type deux pièces, la couche adhésive doit présenter une adhérence plus grande à la piste de la poche qu'à la piste du support.

Ce résultat peut être obtenu de diverses manières connues. D'abord, les natures chimiques des deux pistes peuvent être choisies afin que le matériau de la piste de la poche soit plus compatible avec le matériau de la couche adhésive que le matériau de la piste de support.

Les adhérences relatives de la couche adhésive aux deux pistes peuvent être modulées soit par augmentation d'adhérence à la piste de la poche, soit par réduction d'adhérence à la piste du support. Bien entendu, il est préférable d'obtenir une adhérence élevée et donc d'augmenter l'adhérence à la piste de la poche.

L'augmentation d'adhérence peut être obtenue par un traitement chimique ou par un traitement physique.

Comme traitement chimique, on peut considérer un traitement chimique de la surface par une réaction de substitution de groupes de surfaces du matériau de la piste ou par une simple attaque chimique.

Comme traitement physique, on peut envisager, de manière connue, l'augmentation de rugosité de la surface de la piste de la poche, par exemple par création d'une microrugosité par divers processus physiques, soit purement mécaniques, soit électriques, par exemple par des décharges par effluves.

Un exemple de traitement de réduction d'adhérence à la piste du support peut être au contraire un lissage de la surface de la piste du support, par exemple par utilisation d'un matériau dont la surface a subi une fusion au moins partielle.

Lorsque l'obtention de cette gradation d'adhérence pose des problèmes, il est possible d'utiliser une feuille intermédiaire. Plus précisément, la couche adhésive à propriétés élastomères peut être collée à une feuille mince avec laquelle elle est très compatible, et cette feuille mince est elle-même collée par un adhésif structurel très mince à la piste de la poche. De tels adhésifs peuvent être ceux qui sont couramment utilisés avec une faible épaisseur entre les pistes des appareillages deux pièces connus.

Comme dans tout raccord collé d'appareillage de stomie de type deux pièces, il est souhaitable que les deux parties de raccord soient positionnées préalablement l'une par rapport à l'autre, avant le collage. A cet effet, il est avantageux que le raccord comprenne un organe de positionnement préalable, par exemple de type décrit dans le document WO-2005/025466.

Il faut noter que l'adhésif structurel de la piste de collage de la poche est habituellement découpé dans une feuille. Il n'est pas rare qu'une telle feuille, par exemple formée par calandrage ou extrusion, présente des variations locales d'épaisseur, souvent d'au moins 10 %. Les valeurs données pour l'épaisseur de la couche d'adhésif structurel de la poche peuvent donc varier de manière correspondante.

### Exemple

Dans un exemple, la piste de la poche est formée de polyéthylène de 450 µm d'épaisseur, ayant une résistance à la traction de 49 N. La piste du support est formée de copolymère d'éthylène et d'acétate de vinyle de 350 µm d'épaisseur, ayant une résistance à la traction de 38 N. Sa limite élastique est de 20 N.

Le matériau de la couche d'adhésif structurel à propriétés élastomères est un copolymère styrène-isoprène-styrène ayant une dureté Shore A inférieure à 8, un module de Young inférieur à 0,06 MPa, et une épaisseur de 0,9 mm.

La surface de la piste de la poche a subi un traitement par effluves, de sorte qu'elle présente une force de séparation par pelage nettement supérieure à 38 N, alors que la force de séparation par pelage de la piste du support est de l'ordre de 23 N.

Dans une variante, le matériau de la couche d'adhésif structurel à propriétés élastomères est un polymère d'uréthanne (PUR) ayant une dureté Shore A inférieure à 15, un module de Young inférieur à 0,05 MPa, et une épaisseur de 0,9 mm.

## Revendications

1. Raccord pour appareillage adhésif de stomie, du type deux pièces, comprenant un support muni d'un étrier de fixation à la peau d'un patient et d'une piste de collage, et une poche munie d'une piste de collage portant un adhésif,
**caractérisé en ce que** l'adhésif de la piste de collage de la poche est une couche d'un adhésif structurel ayant une épaisseur d'au moins 0,5 mm.

2. Raccord selon la revendication 1, **caractérisé en ce que** la couche d'adhésif structurel de la poche forme une masse adhésive élastomère.

3. Raccord selon la revendication 2, **caractérisé en ce que** l'adhésif de la couche d'adhésif structurel contient un polymère choisi parmi les polymères d'uréthanne et les copolymères séquencés à séquences triples.

4. Raccord selon la revendication 3, **caractérisé en ce que** le copolymère séquencé est choisi parmi les copolymères styrène-isoprène-styrène, styrène-butadiène-styrène, styrène-éthylène-butadiène-styrène, et styrène-isoprène-butadiène-styrène.

5. Raccord selon l'une quelconque des revendications précédentes, **caractérisé en ce que** la piste de la poche a sa surface portant l'adhésif qui a subi un traitement d'augmentation d'adhérence.

6. Raccord selon la revendication 5, **caractérisé en ce que** le traitement physique d'augmentation d'adhérence est un traitement d'augmentation de la rugosité.

7. Raccord selon l'une quelconque des revendications 1 à 4, **caractérisé en ce que** la couche adhésive est collée sur une feuille intermédiaire de support elle-même collée à la piste de la poche.

8. Raccord selon l'une quelconque des revendications précédentes, **caractérisé en ce qu'**il comporte un organe de positionnement préalable de la piste de la poche par rapport à la piste du support.

9. Raccord selon la revendication 8, **caractérisé en ce que** l'organe de positionnement préalable comprend des
organes de coopération disposés à l'extérieur des pistes du support et de la poche.

10. Raccord selon la revendication 8, **caractérisé en ce que** l'organe de positionnement préalable est disposé à l'intérieur des pistes de la poche et du support, et la couche adhésive de la piste de la poche porte une feuille protectrice formée de deux parties au moins pliées sur elles-mêmes.

## Claims

1. A connector for a stoma adhesive device, of the two-piece type, comprising a support equipped with a bracket for fixing to the skin of a patient and with a gluing channel, and a bag provided with a gluing channel that carries an adhesive,
**characterised in that** the adhesive of the gluing channel of the bag is a layer of a structural adhesive having a thickness of at least 0.5 mm.

2. A connector according to claim 1, **characterised in that** the structural adhesive layer of the bag forms an elastomeric adhesive composition.

3. A connector according to claim 2, **characterised in that** the adhesive of the structural adhesive layer contains a polymer chosen from urethane polymers and three-sequence copolymers.

4. A connector according to claim 3, **characterised in that** the sequence copolymer is chosen from styrene-isoprene-styrene, styrenebutadiene-styrene, styrene-ethylene-butadiene-styrene, and styrene-isoprene-butadiene-styrene copolymers.

5. A connector according to any of the preceding claims, **characterised in that**, in the channel of the bag, its surface carrying the adhesive has undergone a treatment to increase adherence.

6. A connector according to claim 5, **characterised in that** the physical treatment to increase adherence is a treatment to increase the surface roughness.

7. A connector according to any of claims 1 to 4, **characterised in that** the adhesive layer is bonded to an intermediate support sheet, itself bonded to the channel of the bag.

8. A connector according to any of the preceding claims, **characterised in that** it comprises a member for firstly positioning the channel of the bag relative to the channel of the support.

9. A connector according to claim 8, **characterised in that** the member for firstly positioning comprises cooperation members arranged outside of the channels of the support and the bag.

10. A connector according to claim 8, **characterised in that** the member for firstly positioning is arranged inside the channels of the bag and the support, and the adhesive layer of the channel of the bag carries a protective sheet formed of two parts at least folded over themselves.

## Patentansprüche

1. Verbindung für eine Stomavorrichtung und -gerät mit einem Klebstoff-Befestigungssystem, zweiteilige Art, wobei sie eine Halterung, die mit einem auf dem Patientenhaut zu verbindendem Bügel und einer Haftbahn versehen ist, aufweist und wobei sie einen Beutel, der mit einer Haftbahn, die einen Haftmittel trägt, aufweist,
**dadurch gekennzeichnet, dass** das Haftmittel der Haftbahn des Beutels, eine Schicht eines strukturellen Haftmittels ist, die eine Dicke von mindestens 0.5 mm hat.

2. Verbindung nach Anspruch 1, **dadurch gekennzeichnet, dass** die Schicht des strukturellen Haftmittels des Beutels eine Elastomere haftende Masse bildet.

3. Verbindung nach Anspruch 2, **dadurch gekennzeichnet, dass** das Haftmittel der Schicht des strukturellen Haftmittels, ein Polymer ausgewählt aus Urethan Polymeren und dreifach sequenzierten Blockcopolymeren beinhaltet.

4. Verbindung nach Anspruch 3, **dadurch gekennzeichnet, dass** das Blockcopolymer aus Styrol-Isopren-Styrol, StyrolButadien-Styrol, Styrol-Äthylen-Butadien-Styrol und Styrol-Isopren-Butadien-Styrol Copolymeren ausgewählt ist.

5. Verbindung nach einem beliebigen der vorstehenden Ansprüche, **dadurch gekennzeichnet, dass** der Beutelbahn seine Haftmittel tragende Oberfläche, die haftverstärkend behandelt wurde, besitzt.

6. Verbindung nach Anspruch 5, **dadurch gekennzeichnet, dass** die haftverstärkende physische Behandlung, eine Rauheit verstärkende Behandlung ist.

7. Verbindung nach einem beliebigen der vorstehenden Ansprüche 1 bis 4, **dadurch gekennzeichnet, dass** die Haftmittel-Schicht auf eine tragende Zwischenfolie gehaftet ist, die ebenfalls an die Beutelbahn gehaftet ist.

8. Verbindung nach einem beliebigen der vorstehenden Ansprüche, **dadurch gekennzeichnet, dass** es bezogen auf die Bahn der Halterung, einen vor-Positionierungskörper der Beutelbahn aufweist.

9. Verbindung nach Anspruch 8, **dadurch gekennzeichnet, dass** der vor-Positionierungskörper zusammenwirkende Körper aus den Bahnen der Halterung und des Beutels angeordnet, aufweist.

10. Verbindung nach Anspruch 8, **dadurch gekennzeichnet, dass** der vor-Positionierungskörper im inneren der Bahnen des Beutels und der Halterung angeordnet ist und, dass der Haftmittel-Schicht der Beutelbahn eine Schutzfolie trägt, von zwei mindestens in sich gebogene Teile gebildet.
